# EUROPEAN PATENT APPLICATION

(11) **EP 3 950 675 A1**
(43) Date of publication of application: **09.02.2022**
(21) Application number: 20778856.3
(22) Date of filing: 22.01.2020
(51) Int. Cl.: C07D 209/10, C07F 5/02, C09K 11/06, C09B 23/08

(54) **FLUORESCENT COMPOUND, PREPARATION METHOD THEREFOR, AND USE THEREOF**

(30) Priority: 26.03.2019 CN 201910230780
(71) Applicant: Neuboron Medtech Ltd., Nanjing, Jiangsu 211112 (CN)
(72) Inventor: HE, Jing, Nanjing, Jiangsu 211112 (CN); LI, Shi-hong, Nanjing, Jiangsu 211112 (CN); LIU, Yuan-hao, Nanjing, Jiangsu 211112 (CN)
(74) Representative: Gunzelmann, Rainer
(86) International application number: PCT/CN2020/073720
(87) International publication number: WO 2020/192271

(57) **Abstract**

Disclosed is a fluorescent compound as represented by general formula I, or a salt, an enantiomer, a diastereomer, a tautomer, a solvate or a polymorph thereof, having the structure (I); wherein m and n are each an integer between 0-10; and Y₁ and Y₂ are each independently selected from the group of hydrogen, phenyl, hydroxyl, carboxyl, an ester group, a boric acid group, a borate group, and a 3 to 7 membered ring substituted with one or more boric acid groups or borate groups, and at least one of Y₁ and Y₂ is a boron-containing group. The compound has the characteristics of a high fluorescence intensity and a high sensitivity.

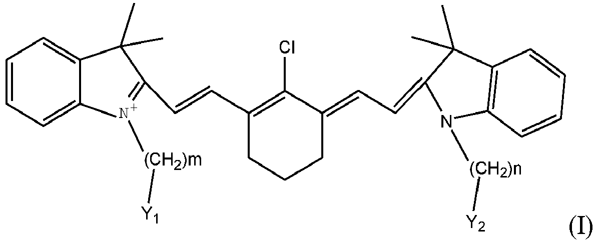

## Description

### BACKGROUND

### Technical Field

The present invention relates to the field of chemical dyes, and in particular, to a fluorescent compound, and a preparation method and use for the same.

### Related Art

Having a large molar extinction coefficient, good fluorescence properties, increased fluorescence efficiency after being combined with a matrix, and a large tunable range of maximum absorption wavelength, cyanine dyes are widely used in various applications, for example, spectral sensitization, infrared laser dyes, optical disc recording media, LB films, electronic photography, optical nonlinear materials, solar cells and trace metal ion detection.

Among other cyanine dyes, the near infrared heptamethine cyanine dye, with a maximum absorption and emission wavelength in a near infrared region between 600 nm and 900 nm, has significant advantages in the analysis of some samples.

### SUMMARY

The present invention provides a fluorescent compound, and a preparation method and use for the same. The fluorescent compound may be used as a fluorescent dye, which has an excitation wavelength in a near infrared region of about 800 nm.

According to a first aspect of the present invention, provided is a fluorescent compound as represented by general formula I, or a salt, an enantiomer, a diastereomer, a tautomer, a solvate or a polymorph thereof, having a structure 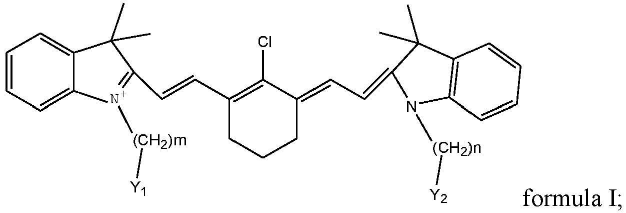 wherein m and n are each independently an integer between 0 and 10; and Y₁ and Y₂ are each independently selected from the group consisting of hydrogen, phenyl, hydroxyl, carboxyl, an ester group, a boric acid group, a borate group, and a 3 to 7 membered ring substituted by one or more boric acid groups or borate groups, and at least one of Y₁ and Y₂ is a boron-containing group.

In another preferred example, Y₁ and Y₂ are each independently selected from the group consisting of hydrogen, phenyl, a boric acid group, a borate group, and 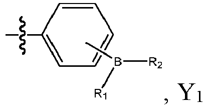 and Y₂ are not both hydrogens, and Y₁ and Y₂ are not both phenyls.

In a further preferred example, m and n are each independently an integer between 0 and 5.

In still another preferred example, m = n; and more preferably, m = n = 0 or m = n = 1.

In another preferred example, R₁ and R₂ are each independently hydroxyl, or taken together with the boron atom to which they are attached, represent a group that can be hydrolyzed into boric acid; and preferably, R₁ and R₂ are each independently hydroxyl, or taken together with the boron atom to which they are attached, form a 5 to 8 membered ring that can be hydrolyzed into boric acid.

In still another preferred example, -BR₁R₂ is a borate group or a boric acid group.

In another preferred example, the salt has a structure of formula II 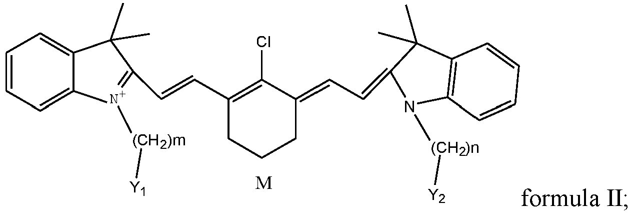 where M represents an anion that forms a salt with N in the formula II.

In another preferred example, M comprises iodide, chloride, bromide, alkylsulfonate, tetrafluoroborate, sulfate, nitrate, phosphate, perchlorate, formate, acetate, or phenylalanine ions; and preferably, M comprises iodide, chloride, and bromide.

In a further preferred example, Y₁ and Y₂ are each independently hydrogen, phenyl, or

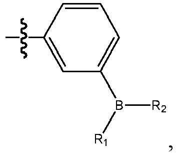

Y₁ and Y₂ are not both hydrogens, and Y₁ and Y₂ are not both phenyls.

In still another preferred example, m and n are each independently an integer between 0 and 5.

In another preferred example, m = n; and more preferably, m = n = 0 or m = n = 1.

In another preferred example, R₁ and R₂ are each independently hydroxyl, or taken together with the boron atom to which they are attached, represent a group that can be hydrolyzed into boric acid, or preferably, taken together with the boron atom to which they are attached, form a 5 to 8 membered ring that can be hydrolyzed into boric acid.

In another preferred example, -BR₁R₂ is a borate group or a boric acid group.

In another preferred example, m = n = 1, and Y₂ is phenyl.

In another preferred example, Y₁ is 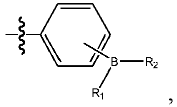 and more preferably is

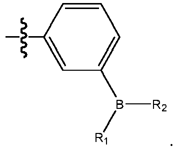

In another preferred example, -BR₁R₂ is a borate group or a boric acid group.

In yet another preferred example, B is ¹⁰B.

In another preferred example, B is ¹⁰B with a purity of greater than or equal to 95%.

According to a second aspect of the present invention, a method for preparing the compound according to the first aspect is provided, the method comprising a step of using a compound of formula c, wherein the compound of formula c has a structure 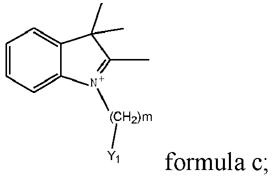 where m and Y₁ are defined as described in the first aspect.

In another preferred example, m is a positive integer between 0 and 5; and Y₁ is

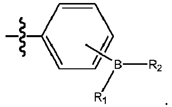

In still another preferred example, Y₁ is

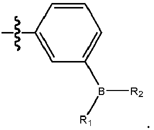

In another preferred example, R₁ and R₂ are each independently hydroxyl, or taken together with the boron atom to which they are attached, represent a group that can be hydrolyzed into boric acid, or preferably, taken together with the boron atom to which they are attached, form a 5 to 8 membered ring that can be hydrolyzed into boric acid.

In another preferred example, m is 0 or 1.

In another preferred example, the step comprises: (i) reacting a compound of formula a with a compound of formula b, to obtain the compound of formula c 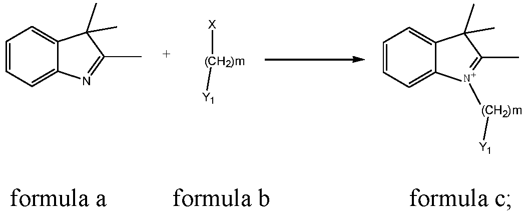 where X is a halogen, and m and Y₁ are as defined in the first aspect.

In another preferred example, X is chlorine, bromine or iodine.

In another preferred example, the step (i) comprises a step of reacting the compound of formula a with the compound of formula b in a first inert solvent, to obtain the compound of formula c.

In a further preferred example, the step (i) is performed under the protection of an inert gas.

In another preferred example, the step (i) is performed at a reflux temperature.

In still another preferred example, the compound of formula a is reacted with the compound of formula b under a microwave condition.

In another preferred example, the method further comprises, after the step (i), (ii) reacting the compound of formula c with a compound of formula c-1, and a compound of formula d, to obtain the compound of formula I, wherein Y₁, m, n, and Y₂ are as defined in the first aspect; 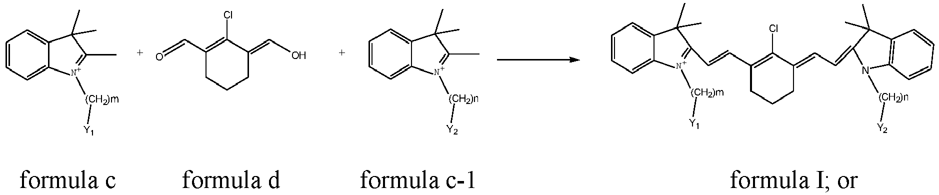 reacting the compound of formula c with the compound of formula d, to obtain the compound of formula I

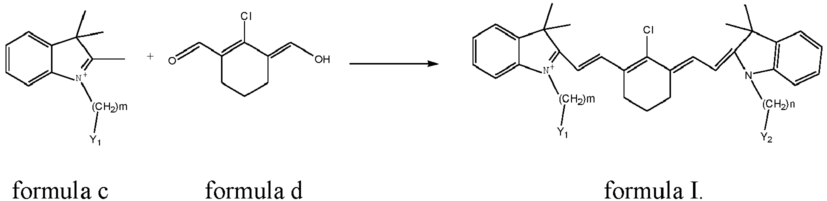

In another preferred example, the compound of formula c-1 is synthesized by the same method as that of the compound of formula c.

In still another preferred example, the compound of formula c-1 is the same as the compound of formula c.

In another preferred example, Y₁ and Y₂ are each independently selected from the group consisting of hydrogen, phenyl, a boric acid group, a borate group, and 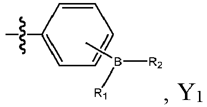 and Y₂ are not both hydrogens, and Y₁ and Y₂ are not both phenyls.

In another preferred example, m and n are each independently an integer between 0 and 5.

In another preferred example, m = n; and more preferably, m = n = 0 or m = n = 1.

In another preferred example, R₁ and R₂ are each independently hydroxyl, or taken together with the boron atom to which they are attached, represent a group that can be hydrolyzed into boric acid.

In another preferred example, -BR₁R₂ is a borate group or a boric acid group.

In another preferred example, Y₂ = Y₁.

In another preferred example, Y₁ is 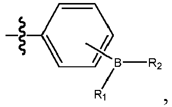 and Y₂ = Y₁.

In a further preferred example, Y₁ is 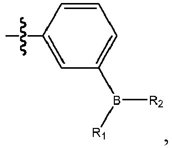 and Y₂ = Y₁.

In another preferred example, Y₂ is phenyl.

In another preferred example, the step (ii) further comprises a step of reacting the compound of formula c with the compound of formula d in a second inert solvent, to obtain the compound of formula I.

In another preferred example, the step (ii) is performed under the protection of an inert gas.

In another preferred example, the step (ii) is performed at a temperature between 30°C and 100°C.

In another preferred example, the step (ii) further comprises a step of adding a base in the second inert solvent.

In another preferred example, the base is an alkali metal salt or an alkali metal hydride, where the alkali metal comprises lithium, sodium, potassium, calcium, magnesium, and cesium.

In another preferred example, the alkali metal salt comprises alkali metal hydroxides and alkali metal organic acid salts.

In another preferred example, the first inert solvent and the second inert solvent are each independently selected from the group consisting of methanol, ethanol, isopropanol, ethylene glycol, N-methyl pyrrolidinone (NMP), dimethyl sulfoxide, tetrahydrofuran, toluene, benzene, dichloromethane, trichloromethane, tetrachloromethane, 1, 2-dichloroethane, acetonitrile, N, N-dimethylformamide (DMF), N, N-dimethylacetamide, dioxane, bis(2-dimethylaminoethyl) ether, or a combination thereof.

In another preferred example, the compound of formula c has a structure of

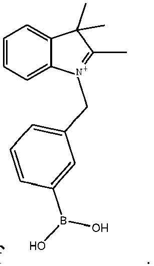

A third aspect of the present invention provides a use of the fluorescent compound, or the salt, the enantiomer, the diastereomer, the tautomer, the solvate or the polymorph thereof according to the first aspect of the present invention, which is used as a fluorescent dye.

It should be understood that within the scope of the present invention, the above-mentioned technical features of the present invention and those described in detail in the following (such as Examples) may be combined with each other, to form new or preferred technical solutions. For the sake of brevity, details are not described herein again.

### BRIEF DESCRIPTION OF THE DRAWINGS

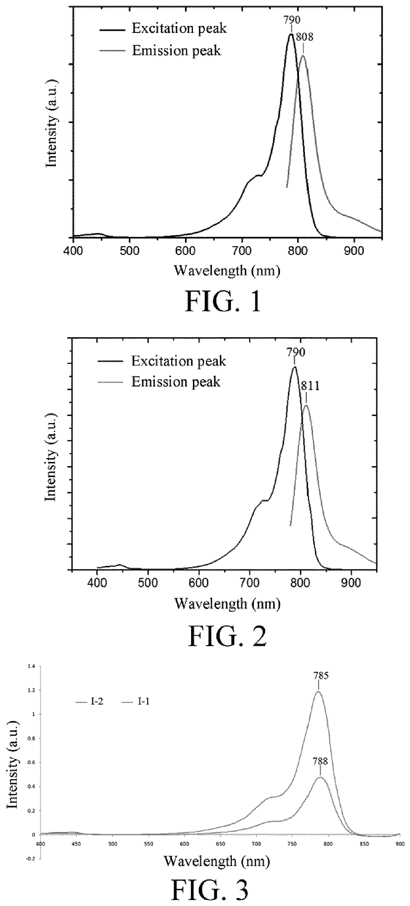

FIG. 1 is a fluorescence spectrum of compound 1-1 in Example 4 at room temperature;
FIG. 2 is a fluorescence spectrum of compound 1-2 in Example 4 at room temperature; and
FIG. 3 is a near-infrared absorption spectrum of compound 1-1 and compound 1-2 in Example 4 in ethanol solution.

### Terminology

Unless otherwise defined, all technical and scientific terms in this specification have the same meanings as that usually understood by a person skilled in the art to which the claimed subject belongs. Unless otherwise stated, all patents, patent applications, and publications cited in the context of the present invention are incorporated herein by reference.

It should be understood that the foregoing brief description and the following detailed description are exemplary and used only for explaining, and are not intended to limit the subject of the present invention. In this application, unless otherwise specifically stated, when a singular form is used, a plural form is also included. It should be noted that, unless otherwise clearly stated in the context, singular forms used in the specification and the claims include plural referents. It should also be noted that, unless otherwise stated, "or", and "alternatively" represent "and/or". In addition, the term "comprise" and other forms, for example, "include", "contain" and "have" are not limiting.

Section titles in this specification are only used for the purpose of organizing the text, and should not be explained as limitations to the subject. All documents or parts of the documents cited in this application, including but are not limited to patents, patent applications, articles, books, operating manuals and papers, are incorporated herein by reference in their entirety.

In addition to the above, when used in the specification and claims of this application, unless otherwise specifically noted, the following terms have meanings shown as follows.

In this application, "B" refers to a boron element, including a radioactive and a nonradioactive boron element, preferably ¹⁰B; and "boric acid group" refers to a -B(OH)₂ group. "3 to 7 membered ring" is a saturated or an unsaturated carbocyclic ring or a heteroatom-containing ring having 3 to 7 carbon atoms, and preferably is an aromatic ring. A "boron-containing group" refers to the group containing a boron atom in this specification.

In this application, "optional" or "optionally" represents that the following described event or condition may happen or may not happen, and the description comprises both the situations where the event or the condition happens and the situations where the event or the condition does not happen. For example, an "optionally substituted aryl group" represents an aryl group that is substituted or unsubstituted, and the description comprises both the substituted aryl group and the unsubstituted aryl group.

A "stereoisomer" refers to a compound composed of the same atoms bonded through the same bonds, but having different three-dimensional structures. The present invention will include various stereoisomers and mixtures thereof.

When a compound of the present invention contains an olefinic double bond, the compound of the present invention is intended to comprise an E- and Z-geometric isomer, unless otherwise stated.

A "tautomer" refers to an isomer formed by transferring a proton from an atom of a molecule to another atom of the same molecule. All tautomer forms of the compound of the present invention are contained in the scope of the present invention.

The compound or the salt thereof of the present invention may contain one or more chiral carbon atoms, and thus can generate enantiomers, diastereomers and stereoisomers thereof. Each of the chiral carbon atoms can be defined as (R)- or (S)- based on stereochemistry. The present invention is intended to comprise all possible isomers, as well as racemates and optically pure forms thereof. For the preparation of the compound of the present invention, the racemates, the diastereomers or the enantiomers may be selected as raw materials or intermediates. An optically active isomer may be prepared by using chiral synthons or chiral reagents, or resolved by using conventional techniques, for example, by using crystallization and chiral chromatography.

In this application, the term "salt" comprises acid addition salts and base addition salts. The "acid addition salts" refer to salts that can retain the biological activities of free alkali without other side effects, and which are formed with inorganic acids or organic acids. Inorganic acid salts include but are not limited to hydrochloride, hydrobromide, sulfate, nitrate, phosphate and the like; and organic acid salts include but are not limited to formate, acetate, 2,2-dichloroacetate, trifluoroacetate, propionate, caproate, caprylate, caprate, undecylenate, glycollate, gluconate, lactate, sebacate, adipate, glutarate, malonate, oxalate, maleate, succinate, fumarate, tartrate, citrate, palmitate, stearate, oleate, cinnamate, laurate, malate, glutamate, pyroglutamate, aspartate, benzoate, mesylate, benzenesulfonate, tosilate, alginate, ascorbate, salicylate, 4-aminosalicylates, napadisylate, and the like. The "base addition salts" refer to salts that can be formed with inorganic bases or organic bases. The salts derived from inorganic bases include but are not limited to sodium, potassium, lithium, ammonium, calcium, magnesium, iron, zinc, copper, manganese, and aluminum salts, and the like. Preferably, the inorganic salts are ammonium, sodium, potassium, calcium and magnesium salts. The salts derived from organic bases include but are not limited to the following salts: primary amines, secondary amines, and tertiary amines, substituted amines, comprising natural substituted amines, cyclic amines, and basic ion exchange resins, for example, ammonia, isopropylamine, trimethylamine, diethylamine, triethylamine, tripropylamine, ethanolamine, diethanolamine, triethanolamine, dimethylethanolamine (DMEA), 2-dimethylaminoethanol, 2-ethylaminoethanol, dicyclohexylamine, lysine, arginine, histidine, caffeine, procaine, choline, betaine, ethylenediamine, glucosamine, methylglucamine, theobromine, purine, piperazine, piperidine, N-ethylpiperidine, polyamine resins, and the like. Preferably, the organic bases comprise isopropylamine, diethylamine, ethanolamine, trimethylamine, dicyclohexylamine, choline and caffeine.

"Polymorph" refers to the different solid crystalline phases generated by some compounds of the present invention due to the presence of two or more different molecular arrangements in a solid state. Some compounds of the present invention may exist in more than one crystal forms. The present invention is intended to comprise various crystal forms and mixtures thereof. Generally, crystallization may generate a solvate of the compound of the present invention. The term "solvate" used in the present invention refers to an aggregate comprising one or more molecules of the compound of the present invention and one or more solvent molecules. The solvent may be water, in which case the solvate is a hydrate. Alternatively, the solvent may be an organic solvent. Therefore, the compound of the present invention may exist in the form of hydrates, including monohydrates, dihydrates, semihydrates, sesquihydrates, trihydrates, tetrahydrates, and the like, as well as the corresponding solvation forms. The compound of the present invention may form a true solvate, but in some cases, it may also retain only some amount of water or a mixture of water and some amount of solvent. The compound of the present invention may be reacted in a solvent or be precipitated out or crystallized from a solvent. The solvate of the compound of the present invention is also included in the scope of the present invention.

### Preparation method for a compound of formula I

The present invention provides a method for preparing compounds of formula I and formula II, the method comprising a step of using a compound of formula c, wherein the compound of formula c has a structure 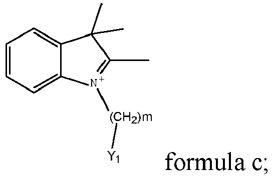 where m and Y₁ are as defined in the first aspect of the present invention.

Preferably, m is an integer between 0 and 5, and more preferably, is 0 or 1; and preferably, Y₁ is 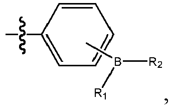 and more preferably is

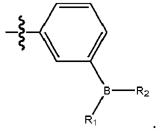

The step comprises: (i) reacting a compound of formula a with a compound of formula b, to obtain the compound of formula c 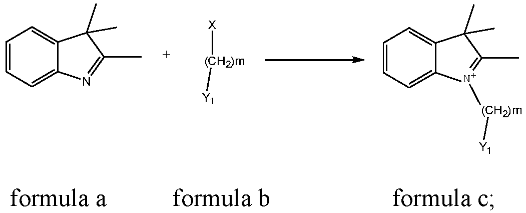 where X is a halogen, and preferably, X is chlorine, bromine or iodine.

In a preferred embodiment, the step (i) comprises a step of reacting the compound of formula a with the compound of formula b in a first inert solvent, to obtain the compound of formula c. Preferably, the step (i) is performed under the protection of an inert gas. Preferably, the step (i) is performed at a reflux temperature. The first inert solvent is not particularly limited, and is preferably selected from the group consisting of methanol, ethanol, isopropanol, ethylene glycol, N-methyl pyrrolidinone (NMP), dimethyl sulfoxide, tetrahydrofuran, toluene, benzene, dichloromethane, trichloromethane, tetrachloromethane, 1,2-dichloroethane, acetonitrile, N, N-dimethylformamide (DMF), N, N-dimethylacetamide, dioxane, bis (2-dimethylaminoethyl) ether, or a combination thereof.

In a preferred embodiment, the step further comprises a step: (ii) reacting the compound of formula c with a compound of formula c-1, and a compound of formula d, to obtain the compound of formula I or the compound of formula II 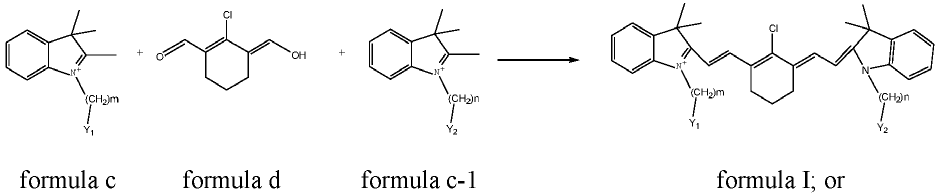 reacting the compound of formula c with the compound of formula d, to obtain the compound of formula I 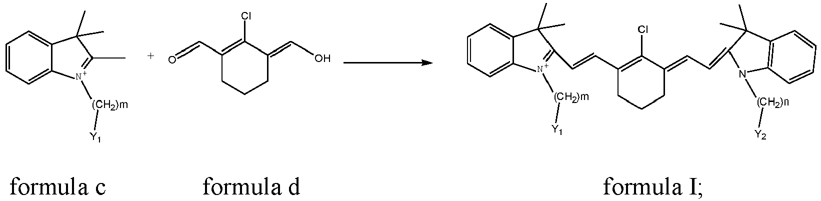 wherein, the compound of formula c-1 is synthesized by the same method as that of the compound of formula c, and the synthetic method of the compound of formula II comprises the synthetic method of the compound of formula I; and m, n, Y₁, and Y₂ are as defined in the first aspect of the present invention. The structure of the compound of formula c-1 and the compound of formula c may be the same or different. In each of the foregoing preparation methods, preferably, Y₁ is 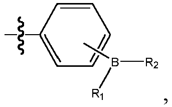 and Y₂ = Y₁; and more preferably, Y₁ is 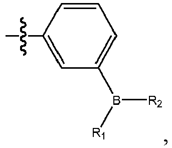 and Y₂ = Y₁. In a preferred embodiment, preferably, m = n, and m and n are both integers from 0 to 5; and more preferably, m = n = 0 or m = n = 1. In a preferred embodiment, Y₁ is 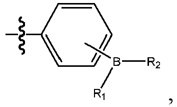 and Y₂ is phenyl; and more preferably, Y₁ is 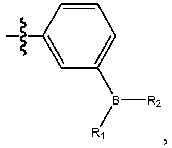 and Y₂ is phenyl. R₁ and R₂ are each independently hydroxyl, or taken together with the boron atom to which they are attached, represent a group that can be hydrolyzed into boric acid. Preferably, -BR₁R₂ is a borate group or a boric acid group.

The step (ii) is performed in the second inert solvent, and preferably performed under the protection of an inert gas. Reaction conditions of the step (ii) are not particularly limited, and preferably, the step (ii) is performed at a temperature between 30°C and 100°C. In a preferred embodiment, the step (ii) further comprises a step of adding a base in the second inert solvent. The base is not particularly limited, and preferably, the base is an alkali metal salt or an alkali metal hydride, where the alkali metal comprises lithium, sodium, potassium, calcium, magnesium, and cesium. Preferably, the alkali metal salt comprises alkali hydroxides and alkali metal organic acid salts. The second inert solvent is also not particularly limited, and is preferably selected from the group consisting of methanol, ethanol, isopropanol, ethylene glycol, N-methyl pyrrolidinone (NMP), dimethyl sulfoxide, tetrahydrofuran, toluene, benzene, dichloromethane, trichloromethane, tetrachloromethane, 1, 2-dichloroethane, acetonitrile, N, N-dimethylformamide (DMF), N, N-dimethylacetamide, dioxane, bis(2-dimethylaminoethyl) ether, or a combination thereof.

It should be noted that in some embodiments, in the step (ii) of the methods for preparing the compound of formula I and the compound of formula II according to the present invention, when the compound of formula c-1 has the same structure as that of the compound of formula c, it is also possible to obtain two or three compounds of formula I and formula II with different structures at the same time, and such cases should also be included in the technical solutions of this application. For example, in Example 4 of this application, compounds 1-1 and 1-2 are obtained at the same time.

For the reaction in each of the steps, reaction temperature may be properly selected according to solvents, starting materials, reagents and the like, and reaction time may also be properly selected according to the reaction temperature, the solvents, the starting materials, the reagents and the like. After the reaction in each of the steps is finished, target compound may be separated and purified from the reaction system by conventional methods, such as filtration, extraction, recrystallization, washing, and slica gel column chromatography. In the case that the next reaction is unaffected, the target compound may also be used in the next reaction directly without separation and purification.

### DETAILED DESCRIPTION

N-substituted side chain is an important part of the molecular structure of dyes. The light stability and fluorescence activity of dye molecules obtained by using different substituents have significant differences, and thus it is crucial to select properly substituted side chains. Through extensive and intensive research, the inventor found a fluorescent compound having structures of formula I and formula II, which has a high fluorescence intensity and a high sensitivity. The present invention is made on this basis.

The following further describes the present invention with reference to the specific examples. It should be understood that the following descriptions are only optimal embodiments of the present invention, and should not be regarded as limitations to the protection scope of the present invention. On the basis of fully understanding the present invention, the experimental methods without specified conditions in the following examples usually follow the conventional conditions, or the conditions suggested by manufacturers. Those skilled in the art can make non-essential changes to the technical solutions of the present invention, and such changes should be regarded to be included within the protection scope of the present invention. Unless otherwise stated, the percentages and parts are percentages by weight and parts by weight.

### <Example 1>

A dry three-necked flask is purged with nitrogen to ensure that the solvents and raw materials are water-free. 180 ml of bis(2-dimethylaminoethyl)ether is added, and then 3.3 g of NaH is added with stirring, followed by 30 g of 3-methyl iodobenzene. Stirring is continued until no gas is substantially evolved. 101 g of n-butyl borate is added to the flask under a nitrogen atmosphere, and cooled to below 0°C. 375 ml of tert-butylmagnesium chloride is added dropwise to the mixture slowly, while the temperature is maintained at 15°C or less. After addition, the resulting mixture was naturally warmed to room temperature. After 3 h of the reaction, TLC shows that the reaction is completed. The mixture is diluted with 1800 ml of a mixed solvent of methyl tert-butyl ether:ethyl acetate (1:1), and quenched with water. The temperature during quenching is not higher than 30°C. To the resulting mixture is added 6 mol/L of hydrochloric acid dropwise to adjust pH to 3-4. The phases are separated. The organic phase is washed with water for four times, and then with brine, and spin dried to obtain a crude product. The crude product is washed with a solvent of petroleum ether:ethyl acetate = 8:1, filtered and dried to obtain 15.7 g of 3-methyl phenylboronic acid with a yield of 83.92% and a purity of 99.02%.

### <Example 2>

To a three-necked flask is added 200 ml of dry CCl₄, 9 g of 3-methyl phenylboronic acid, 1.6 g of benzoyl peroxide, and warmed up to 65°C with stirring under the protection of nitrogen for 5 min, to initiate benzoyl peroxide. 11.64 g N-bromosuccinimide is added in batches. After reflux reaction for overnight, the reaction mixture is cooled down to room temperature, stirred and filtered. The resulting product is washed with a mixed solvent of petroleum ether:ethyl acetate = 8:1 to obtain 13.5 g of 3-bromomethyl phenylboronic acid with a yield of 95.32% and a purity of 91.33%.

### <Example 3>

To a single-necked flask is added 100 ml dry toluene, 9.07 g of 2,3,3-dimethyl-trihydroindole, and 13.5 g 3-bromomethyl phenylboronic acid. The reaction mixture is refluxed under the protection of nitrogen overnight. The resulting mixture is cooled down to room temperature, and filtered to obtain a magenta solid. The solid is washed with ethyl acetate to remove impurities, and dried to give 15 g of 3-bromomethyl phenylboronate indole quaternary ammonium salt with a yield of 89.44% and a purity of 97.33%.

### <Example 4>

To a single-necked flask is added 300 ml of absolute ethyl alcohol, 6.47 g of 3-bromomethyl phenylboronate indole quaternary ammonium salt, 2-chloro-3-hydroxymethylene cyclohexene carboxaldehyde, and 0.15 g of sodium acetate. The mixture is reacted at 70°C for 1 h, and spin dried to remove the solvent. The resulting residue is separated by column chromatography, to obtain fluorescent compounds I-1 and I-2. The fluorescent compound I-1 has a maximum fluorescence emission peak at 808 nm when excited at a wavelength of 790 mm, and has a near infrared absorption peak in ethanol solution at 785 nm. The fluorescent compound 1-2 has a maximum fluorescence emission peak at 811 nm when excited at a wavelength of 790 mm, and has a near infrared absorption peak in ethanol solution at 788 nm.

Compound I-1 ¹H NMR (500 MHz, DMSO-D6):
δ = 1.72-1.74 (14H, 4CH₃, CH₂, m), 2.53-2.54 (4H, 2 CH₂, m), 3.17 (4H, s), 4.04 (2H, s, br), 5.54 (4H, s, br), 6.38 (2H, CH, d = 14.0), 7.26-7.36 (6H, m, ArCH), 7.39 (2H, ArCH, d = 2.8), 7.66-7.73 (6H, ArCH, m), 8.01 (2H, ArCH, s), 8.24 (2H, CH, d = 14.0).

Compound 1-2 ¹H NMR (500 MHz, DMSO-D6):
δ = 1.66-1.73 (14H, 4CH₃, CH2, m), 2.52-2.56 (4H, 2CH₂, m), 3.12 (2H, s), 3.61 (2H, s), 5.50 (4H, s, br), 6.41 (2H, m, CH), 7.31-7.41 (13H, m, ArCH), 7.62-7.73 (4H, ArCH,m), 8.24-8.28 (2H, CH, m).

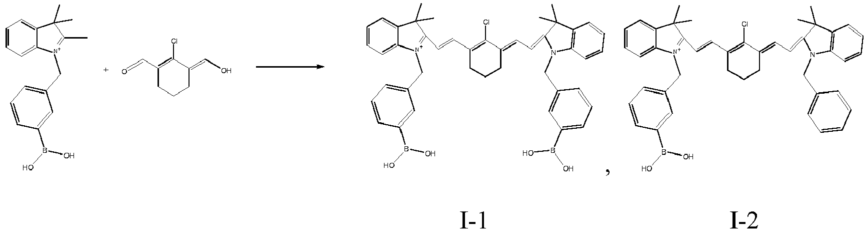

All the references mentioned in the present invention are incorporated herein by references, just as if each of the references is individually incorporated by reference. In addition, it should be understood that, after reading the foregoing teaching of the present invention, those skilled in the art may make various changes or modifications to the present invention, and these equivalent forms also fall within the scope defined by the appended claims of this application.

## Claims

1. A fluorescent compound as represented by general formula I, or a salt, an enantiomer, a diastereomer, a tautomer, a solvate or a polymorph thereof, having a structure 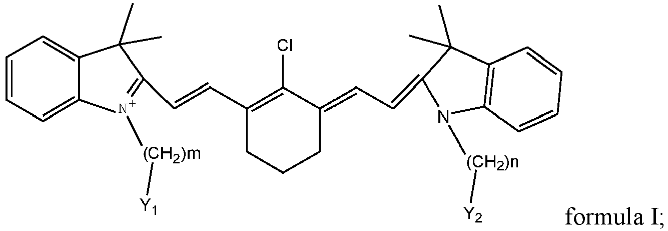 wherein m and n are each independently an integer between 0 and 10; and
Y₁ and Y₂ are each independently selected from the group consisting of hydrogen, phenyl, hydroxyl, carboxyl, an ester group, a boric acid group, a borate group, and a 3 to 7 membered ring substituted by one or more boric acid groups or borate groups, and at least one of Y₁ and Y₂ is a boron-containing group.

2. The fluorescent compound, or the salt, the enantiomer, the diastereomer, the tautomer, the solvate or the polymorph thereof according to claim 1, wherein Y₁ and Y₂ are each independently selected from the group consisting of hydrogen, phenyl, a boric acid group, a borate group, and 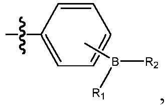 and Y₁ and Y₂ are not both hydrogens, and Y₁ and Y₂ are not both phenyls; and R₁ and R₂ in the formula are each independently hydroxyl, or taken together with the boron atom to which they are attached, represent a group that is hydrolyzable into boric acid.

3. The fluorescent compound, or the salt, the enantiomer, the diastereomer, the tautomer, the solvate or the polymorph thereof according to claim 1, wherein the salt has a structure of formula II 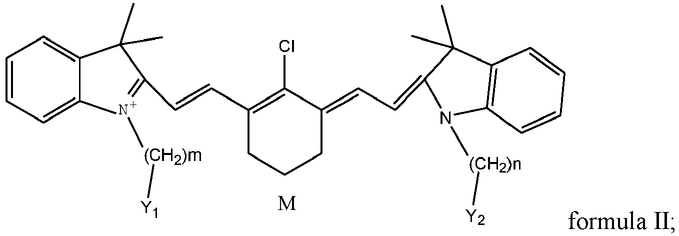 wherein M represents an anion that forms a salt with N in the formula II.

4. The fluorescent compound, or the salt, the enantiomer, the diastereomer, the tautomer, the solvate or the polymorph thereof according to claim 3, wherein M comprises iodide, chloride, bromide, alkylsulfonate, tetrafluoroborate, sulfate, nitrate, phosphate, perchlorate, formate, acetate, or phenylalanine ions.

5. The fluorescent compound, or the salt, the enantiomer, the diastereomer, the tautomer, the solvate or the polymorph thereof according to any one of claims 1 to 3, wherein m and n are each independently an integer between 0 and 5.

6. The fluorescent compound, or the salt, the enantiomer, the diastereomer, the tautomer, the solvate or the polymorph thereof according to claims 1 to 3, having a structure

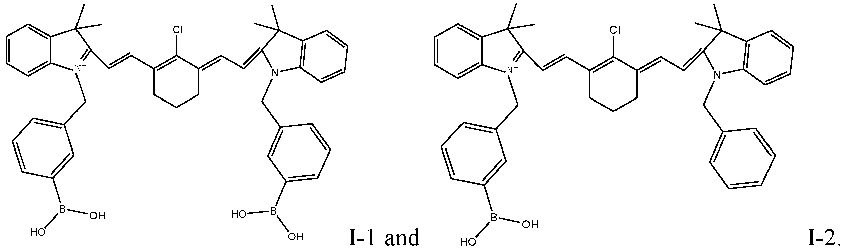

7. The fluorescent compound, or the salt, the enantiomer, the diastereomer, the tautomer, the solvate or the polymorph thereof according to claims 1 to 3, wherein B is ¹⁰B.

8. A method for preparing the fluorescent compound or the salt thereof according to any one of claims 1 to 3, comprising a step of using a compound of formula c, wherein the compound of formula c has a structure 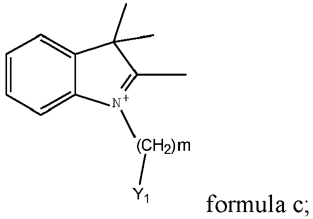 wherein m is an integer between 0 and 10;
Y₁ is selected from the group consisting of hydrogen, phenyl, hydroxyl, carboxyl, an ester group, a boric acid group, a borate group, and a 3 to 7 membered ring substituted by one or more boric acid groups or borate groups; and
preferably, the compound of formula c has a structure of

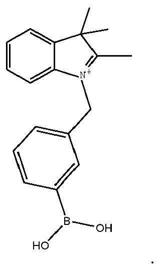

9. The method according to claim 8, further comprising a step: (i) reacting a compound of formula a with a compound of formula b, to obtain the compound of formula c 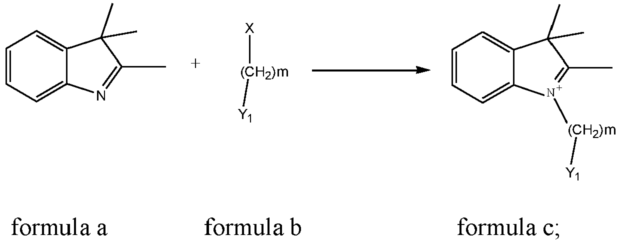 wherein X is a halogen; m is an integer between 0 and 10; and
Y₁ is selected from the group consisting of hydrogen, phenyl, hydroxyl, carboxyl, an ester group, a boric acid group, a borate group, and a 3 to 7 membered ring substituted by one or more boric acid groups or boric acid ester groups.

10. The method according to claim 8, further comprising a step: (ii) reacting the compound of formula c with a compound of formula c-1, and a compound of formula d, to obtain the compound of formula I 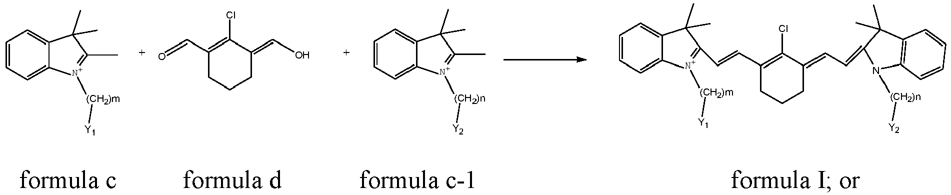 reacting the compound of formula c with the compound of formula d, to obtain the compound of formula I 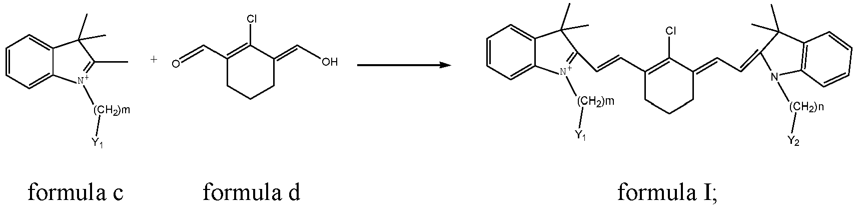 wherein m and n are each an integer between 0 and 10; and
Y₁ and Y₂ are each independently selected from the group consisting of hydrogen, phenyl, hydroxyl, carboxyl, an ester group, a boric acid group, a borate group, and a 3 to 7 membered ring substituted by one or more boric acid groups or borate groups; and at least one of Y₁ and Y₂ is a boron-containing group.

11. The method according to claim 10, wherein the compound of formula c-1 is synthesized by the same method as that of the compound of formula c.

12. The method according to claim 10, wherein the compound of formula c-1 is the same as the compound of formula c.

13. The method according to claims 8 to 10, wherein m is a positive integer between 0 and 5; Y₁ is 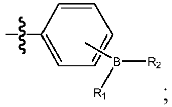 and R₁ and R₂ in the formula are each independently hydroxyl, or taken together with the boron atom to which they are attached, represent a group that is hydrolyzable into boric acid.

14. The method according to claims 8 to 10, wherein B is ¹⁰B.

15. Use of the fluorescent compound, or the salt, the enantiomer, the diastereomer, the tautomer, the solvate or the polymorph thereof according to any one of claims 1 to 3, used as a fluorescent dye.
